## Europäisches Patentamt
## European Patent Office
## Office européen des brevets

(19)

(11) Veröffentlichungsnummer : **0 362 202 B1**

(12) **EUROPÄISCHE PATENTSCHRIFT**

(45) Veröffentlichungstag der Patentschrift :
08.04.92 Patentblatt 92/15

(51) Int. Cl.⁵ : **B01D 15/08,** A61M 1/36,
B01J 20/32

(21) Anmeldenummer : 87907577.8

(22) Anmeldetag : 18.11.87

(86) Internationale Anmeldenummer :
PCT/DE87/00536

(87) Internationale Veröffentlichungsnummer :
WO 88/09202 01.12.88 Gazette 88/26

(54) **MITTEL ZUR HÄMOPERFUSION ODER HÄMOADSORPTION.**

(30) Priorität : 25.05.87 DE 3717951

(43) Veröffentlichungstag der Anmeldung :
11.04.90 Patentblatt 90/15

(45) Bekanntmachung des Hinweises auf die
Patenterteilung :
08.04.92 Patentblatt 92/15

(84) Benannte Vertragsstaaten :
AT CH DE FR GB LI NL SE

(56) Entgegenhaltungen :
EP-A- 0 056 977
DE-A- 2 601 089
GB-A- 2 101 906
Chemical Abstracts, vol. 107, 13 July 1987,
Columbus, Ohio, US; I.P. Andrianova et
al.:"Binding of cholesterin lipoprotein complexes by silica matrixes", abstract no. 12883f

(56) Entgegenhaltungen :
Chemical Abstracts, vol. 96, 12 April 1982,
Columbus, Ohio, US; E.A. Khol et
al.:"Improved high-pressure liquid-chromatographic assay of serum 25-hydroxycholecalci-
ferol and 25-hydroxyergocalciferol after
reverse-phase Sep-Pak C 18 cartridge preparation of sample", pp. 306-307, abstract no.
118345b

(73) Patentinhaber : MURUGAVEL, Shanmugam
Riehlstrasse 6
W-1000 Berlin 19 (DE)

(72) Erfinder : MURUGAVEL, Shanmugam
Riehlstrasse 6
W-1000 Berlin 19 (DE)

(74) Vertreter : Maikowski, Michael, Dipl.-Ing. Dr.
Patentanwälte Maikowski & Ninnemann
Xantener Strasse 10
W-1000 Berlin 15 (DE)

Anmerkung : Innerhalb von neun Monaten nach der Bekanntmachung des Hinweises auf die Erteilung des europäischen Patents kann jedermann beim Europäischen Patentamt gegen das erteilte europä- ische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patent- übereinkommen).

**Beschreibung**

Die Erfindung betrifft Reversed-Phase-Kieselgel als Mittel zur Hämoperfusion oder Hämoadsorption.

Unter Hämoperfusion oder Hämoadsorption versteht man die adsorptive Bindung von im Blut gelösten Substanzen an oberflächenreiche Feststoffe. Mit diesem Verfahren ist es möglich, exogene und endogene Toxine wie zum Beispiel Amtriptylin, Chinin-HCl, Digoxin, Digitoxin und Methaqualon direkt aus dem Blut zu entfernen. Während der Durchströmung sinkt die Konzentration des zu entfernenden Stoffes, des Sorptivs, bei ausreichender Feststoffkapazität und idealen Bindungsbedingungen zwischen Sorptiv und Sorbens praktisch auf null. Bei den bekannten Verfahren werden als Sorbens Aktivlohle und verschiedene Ionenaustauscher bei der Hämoperfusion verwendet. (E. Wetzels, A. Clombi, P. Dittrich, H. J. Gurland, M. Kessel und H. Klinkmann, "Hämodialyse, Peritonealdialyse, Membranplasmapherese und verwandte Verfahren", Springer-Verlag, Berlin 1986, Seiten 66 - 69 und 606 - 608; und H. E. Franz (Hrsg.), "Blutreinigungsverfahren", G. Thieme Verlag, Stuttgart 1985, Seiten 15 und 473 - 477.)

Die bisher verwendeten Adsorbentien sind in mancher Hinsicht nicht zufriedenstellend. Es besteht deshalb ein Bedarf nach neuen Mitteln zur Hämoperfusion oder Hämoadsorption.

Der Erfindung liegt die Aufgabe zu Grunde, neue Mittel für die vorgenannten Zwecke zur Verfügung zu stellen.

Überraschend hat sich gezeigt, daß Silica-Gel ein ausgezeichnetes und reichlich zur Verfügung stehendes Mittel zur Hämoperfusion oder Hämoadsorption ist. Als Silica-Gel oder Kieselgel wird allgemein kolloide dichte oder lockere, geformte oder ungeformte Kieselsäure bezeichnet, die eine feine Porenstruktur und dadurch ein hohes Adsorptionsvermögen aufweist.

Als besonders geeignet hat sich als Silica-Gel ein Reversed-Phase-Kieselgel herausgestellt. In der Reversed-Phase enthält das Silica-Gel an den oberflächlichen Hydroxylgruppen kovalent gebundene octyl- oder octadecylfunktionelle Gruppen. Dadurch wird die Oberfläche im wesentlichen unpolar und hydrophob. Reversed-Phase Silica-Gel und dessen Eigenschaften werden in den Literaturstellen R. C. Dunhy, "A dictionary of chromotography" Macimillan reference books, Seite 160; C. Horvath, "Reversed-phase chromotography", Trends in analytical chemistry, Reference edition 1.1981, Seiten 7 - 8 und K. K. Unger, "Porons silica", Journal of Chromatography Libary, Vol. 16, Elservier Sc. Publ. Co., Amsterdam, Seiten 216 -217, beschrieben. Als besonders geeignet hat sich ein Reversed-Phase-Kieselgel erwiesen, das einen Porendurchmesser von etwa 10 nm bei einer Korngröße von 40 - 60 Micrometer hat. Dieses Silica-Gel ist im wesentlichen unpolar und hydrophob. Der pH-Wert der wäßrigen Suspension beträgt 7.

Der nicht polare Dimethyloctylligand wurde durch eine Siloxan-Brücke an der Oberfläche verankert, wodurch die Oberfläche hydrophob wird. Die Oberfläche wird dadurch nicht vollständig bedeckt. Einige polare Silanol-Gruppen existieren noch. Eine nachträgliche Behandlung mit Tetramethylchlorsäure reduziert die restliche polare Silanolkonzentration. Trotzdem wird eine wahre hydrophobe Oberfläche nie erreicht, auch nicht wenn man octadecylfunktionelle Gruppen anwendet.

Das in den Experimenten verwendete Adsorbens heißt Lichroprep RP-18[R]. Seine physikalischen Eigenschaften sind in Tabelle 1 angegeben. Lichroprep RP-18[R] ist ein Reversed-Phase-Kieselgel der Firma Merck.

## Tabelle 1: Chemisch-physikalische Daten von Lichroprep RP-18[R]

| | |
|---|---|
| Porendurchmesser | 10 nm |
| Korngröße | 40-60 Micrometer |
| Polar/Unpolar | Unpolar |
| Hydrophyl/Hydrophob | Hydrophob |
| pH der wäßrigen Suspension | 7 |

Bei der Herstellung der Reversed-Phase-Kieselgel verwendet man Silica mit durchschnittlichem Porendurchmesser von 6 - 10 nm, wobei Silica zuerst mit Säure gesäubert und anschließend durch Auswaschen mit destilliertem Wasser neutralisiert wird. Anschließend wird Silica über Nacht bei 180° - 200° C getrocknet. Zur Herstellung der Reversed-Phase werden folgende Chlorsilane angewendet:

1. Dimethyldichlorsilan-$Cl_2Si(CH_3)2$
2. Di-n-Butyldichlorsilan-$Cl_2Si(C_4H_9)2$

2

3. Methyl-n-Decyldichlorsilan-$Cl_2Si(CH_3)C_{10}H_{21}$

4. Methyl-n-Octadecyldichlorsilan-$Cl_2Si(CH_3)C_{18}H_{37}$

5. n-Octadecyltrichlorsilan-$Cl_3SiC_{18}H_{37}$

Die folgenden Experimente zeigen die mit Lichroprep RP-18[R] erfolgreich durchgeführten Hämoperfusionen oder Hämoadsorptionen.

## Kartuschenaufbau

Als Minikartusche für den invitro-Versuch wurde ein Plastikröhrchen aus Polypropylen mit SchraubverschlußDeckel (Fa. Sarsteadt) benutzt. Das Röhrchen von 6 cm Höhe und 1,5 cm Durchmesser faßt 8 ml. Im Deckel wurde über ein durchgebohrtes Loch ein Adapter angebracht. Der Adapter diente zum Anschluß des SMA-Flow-rated-Pumpschlauches (Fa. Technicon Instrumental Corporation, Tarrytown, New York). Die Zuleitung zu den Techniconschläuchen wurde aus Infusionssystemen hergestellt. Um das Perfusionsmedium sammeln zu können, wurde das untere Ende des Röhrchens durchbohrt und mit einem Schaumstoffilter versehen. Der Schaumstoffilter bestand aus 4 - 5 untereinander vernetzten Polyäthylenschichten mit Porendurchmessern von 0,2 mm bis 2 mm.

## Trägerlösungen für Pharmaka

Als Trägerlösung für die Pharmaka wurde Humanplasma verwendet, das durch Zentrifugieren mehrerer verfallener Blutkonserven gewonnen wurde.

## Durchführung der Experimente

Drei Gramm Lichroprep RP-18[R] wurden in ein Glasröhrchen mit einer Öffnung am unteren Ende gegeben. Um eine homogene Benetzung des Adsorbens mit Blut/Plasma zu erreichen, wurde dem Adsorbens Äthanol-/Aqua bidest. 1 : 2 zugemischt. Der Inhalt wurde mehrmals kräftig manuell geschüttelt. Das so präparierte Adsorbens wurde in die Kartusche gefüllt. Das Lösungsmittel Äthanol/Aqua bidest. tropfte durch das Loch am unteren Ende des Röhrchens ab. Durch das Abtropfen des Lösungsmittels wurde das Adsorbens homogen und dicht gepackt sedimentiert. Kartusche mit dem Adsorbens wurde dann mit ca. 200 ml Aqua bidest. durchgespült, um das restliche Lösungsmittel auszuwaschen.

Die Pharmaka, verwendet wurden Amitriptylin, Chinin, Digoxin, Digitoxin, Methaqualon, Phenobarbital und Phenytoin, wurden in toxischer Konzentration in jeweils einem Liter Plasma aufgelöst und mehrmals kräftig manuell geschüttelt.

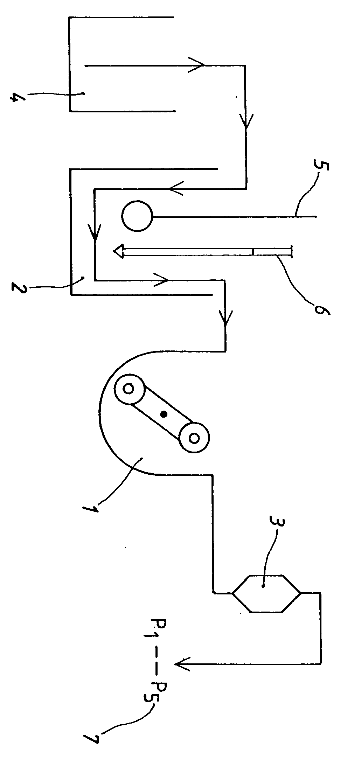

Die Figur 1 zeigt die schematische Darstellung für die Experimente.

Wie ersichtlich, werden die Technicon-Schläuche über eine Präzisions-Rollerpumpe 1 (Auto analyser proportioning pump, Technicon Instrumental Corporation, Tarrytown, New York) eingespannt. Die Zuleitungsschläuche aus dem Plasma 4 wurden durch ein Wasserbad 2 mit Heizkörper 5 und Thermometer 6 von 37° C geführt.

Um die Standarabweichung bestimmen zu können, wurden pro Pharmakon jeweils fünf Experimente mit fünf parallel durchströmten Kartuschen durchgeführt. Diese Kartuschen 3 wurden mit der Plasma-Pharmakon-Lösung mit einer Flußgeschwindigkeit von 1 ml/Min. perfundiert. Die Gesamtlaufzeit betrug 180 Minuten. Damit wurde jede Kartusche von 180 ml Lösung durchströmt.

Zur Bestimmung der Konzentrationen der eingesetzten Pharmaka wurden Proben vor ($P_0$) und nach dreistündiger Perfusion ($P_1$ bis $P_5$ - von den fünf parallel durchströmten Kartuschen) 7 entnommen.

Es wurden zusätzliche Perfusionsexperimente mit heparinisiertem Frischblut zur Bestimmung der Biokompatibilität bezüglich der Gerinnungsparameter und der zellulären Bestandteile des Blutes sowie mit reinem Plasma (ohne Pharmaka) zur Bestimmung der Biokompatibilität bezüglich der nichtzellulären Bestandteile des Blutes durchgeführt.

Dabei wurden folgende Parameter untersucht:

Elektrophorese, Gerinnungsparameter (TPZ, PTT, TZ und Fibrinogen), Kolloidosmotischer Druck, pH, $HCO_3$, Erythrozytenzahl, Hämoglobin, Hämatokrit, mittleres korpuskuläres Volumen, mittlere korpuskuläre Hämoglobinkonzentration, Leukozytenzahl, Thrombozytenzahl sowie Natrium, Kalium, Chlorid, Kalzium, anorganisches Phosphat, Eisen, Glucose, Harnstoff, Kreatinin, Harnsäure, Gesamteiweiß, Albumin, Bilirubin, Cholesterin und Triglyzeride.

### Bestimmungsmethoden

#### 1. Amitriptylin

Amitriptylin wurde mit einem Enzym-Immunoassay (Fa. Syva, Darmstadt) mit einem dafür sensitiven Antiserum bestimmt. Die Linearität des Meßverfahrens liegt im Bereich von 0,2 bis 1 µg/ml. Eine Verdünnung der Proben wurde mit Negativserum vorgenommen. Der untere Meßbereich bei diesem Verfahren liegt bei 0,1 µg/ml, der obere bei 1 µg/ml.

#### 2. Chinin-HCl

Die Proben wurden mit Trichloressigsäure enteiweißt, der klare Überstand mit Natronlauge auf pH 12 eingestellt und dann spektrophotometrisch bei einer Wellenlänge von 250 nm gemessen. Das Verfahren mißt linear im Bereich von 0,6 bis 20 µg/ml.

#### 3. Digoxin

Digoxin wurde mit einem Radioimmunassay (Becton Dickinson Kit) bestimmt.

#### 4. Digitoxin

Digitoxin wurde ebenfalls mit Radioimmunoassay (Coat-A-Count - Digitoxin-Diagnostic Products Corporation) untersucht.

#### 5. Methaqualon

Die Proben wurden mit Pufferlösung auf pH 10 eingestellt und mit Dichlormethan Isopropanol 9 : 1 extrahiert. Das Lösungsmittel wurde entfernt und der Rückstand in 100 ml Methanol aufgenommen. Dann wurde mit einer OV-17 Säule bei 290° C gaschromatographiert. Der lineare Meßbereich reicht von 0,1 bis 20 µg/ml.

#### 6. Phenobarbital

Phenobarbital wurde spektralphotometrisch nach der Methode von Zak mit einem Spektralphotometer Pye-Unicam PU 8800 UV/vis der Firma Philips bestimmt. Das Plasma wurde bei pH 7,4 mit Chloroform extrahiert. Ein aliquoter Teil der abgetrennten Chloroformphase wurde mit Natronlauge reextrahiert. Dabei gehen die Barbiturate in die Natronlauge über und können durch anschließende spektralphotometrische Messung nachgewiesen werden. Das Adsorbtionsmaximum der Barbiturate liegt in 0,5 N Natronlauge bei 253 bis 255 nm (Clin. Chem. 1973, Seite 615).

#### 7. Phenytoin

Die Bestimmung der Phenytoinkonzentration erfolgte mit der Fluoreszenz-Polarisations-Immunoassay Technik (TDx-System, Fa. Abbott-Diagnostics Division, Wiesbaden).

### Ergebnisse

#### Pharmaka

In der Tabelle 2 sind die Meßwerte für die untersuchten Pharmaka wiedergegeben.
Pharmaka wiedergegeben.
Es bedeutet:
$P_0$ Konzentration vor der Perfusion
$P_1$ - $P_5$ Konzentration nach der Perfusion
P Mittelwert der Konzentration nach der Perfusion
SD Standardabweichung
* Standardabweichung der Untersuchungsmethodik

Clearance : $\dfrac{P_0 - (P_1 \ldots P_5)}{P_0}$ x Flow (ml/Min).

wobei der Flow 1 ml/Min. betrug.

*Tabelle 2:*

In vitro Clearancewerte für Lichroprep RP - 18[R]

| Pharmaka | Konz. | $P_0$ | $P_1$ | $P_2$ | $P_3$ | $P_4$ | $P_5$ | $\bar{P}$ | SD | Durchschnittliche Clearance (ml / Min.) | Elimination in % der Ausgangs-konz. |
|---|---|---|---|---|---|---|---|---|---|---|---|
| Amitryptilin | µg / ml | 10,6 | <0,1 | <0,1 | <0,1 | <0,1 | <0,1 | <0,1 | ± 0,05* | 0,999 | 99,06 |
| Chinin | µg / ml | 8,6 | 1,5 | 1,7 | 1,2 | 0,0 | 1,5 | 0,96 | 0,08 | 0,888 | 88,84 |
| Digoxin | ng / ml | 4,0 | <0,15 | <0,15 | <0,15 | <0,15 | <0,15 | <0,15 | ± 0,05* | 0,96 | 96,33 |
| Digitoxin | ng / ml | 46,9 | <2,0 | <2,0 | <2,0 | <2,0 | <2,0 | <2,0 | ± 0,41* | 0,96 | 95,74 |
| Methaqualon | µg / ml | 17,2 | 0,2 | 0,1 | 0,1 | 0,1 | 0,1 | 0,12 | 0,04 | 0,99 | 93,30 |
| Phenobarbital | mg/dl | 96,4 | 1,8 | 2,2 | 1,8 | 1,8 | 1,82 | 1,88 | 0,23 | 0,979 | 98,04 |
| Phenytoin | µg/ml | 50,5 | 0,69 | 0,02 | 0,03 | 0,27 | 0,33 | 0,26 | 0,27 | 0,995 | 99,58 |

EP 0 362 202 B1

### a) Amitriptylin

Bei einer Ausgangskonzentration von 10,6 g/ml lag die Konzentration nach der Perfusion unterhalb der Nachweisgrenze von 0,1 ± 0,05 µg/ml.

Der berechnete durchschnittliche Clearancewert betrug 0,99 ml/Min. bei einem Flow von 1 ml/Min. Die prozentuale durchschnittliche Elimination lag bei 99% des gesamten Amitriptylin.

### b) Chinin-CHI

Die Ausgangskonzentration $P_0$ betrug 8,6 µg/ml. Der Mittelwert p der Konzentrationen nach der Perfusion lag bei 0,96 ± 0,08 µg/ml.

Bei einem Flow von 1 ml/Min. errechnete sich eine durch schnittliche Clearance von 0,89 ml/Min., sowie eine durchschnittliche prozentuale Elimination von 88,84 % des Chinins.

### c) Digoxin

Die Ausgangskonzentration $P_0$ betrug 4,0 ng/ml. Nach der Perfusion war kein Digoxin mehr nachweisbar. Die untere nachweisbare Grenze liegt bei 0,147 ng/ml. Damit betrug die durchschnittliche Clearance 0,96 ml/Min. bei einem Flow von 1 ml/Min. und die durchschnittliche prozentuale Elimination 96,33 %.

### d) Digitoxin

Die Ausgangskonzentration $P_0$ betrug 46,9 ng/ml. Nach der Perfusion war kein Digitoxin mehr nachweisbar. Die untere nachweisbare Grenze liegt bei < 2 ng/ml. Bei einem Flow von 1 ml/Min. bestimmte sich die durchschnittliche Clearance zu 0,96 ml/Min. und die durchschnittliche prozentuale Elimination zu 95,74 %.

### e) Methaqualon

Bei einer Ausgangskonzentration $P_0$ von 17,2 µg/ml lagen die Konzentrationen nach der Perfusion im Mittel bei 0,12 ± 0,04 µg/ml, was einer durchschnittlichen Clearance von 0,99 ml/Min. bei einem Flow von 1 ml/Min. beziehungsweise einer durchschnittlichen prozentualen Elimination von 93,30 % entspricht.

### f) Phenobarbital

Bei einer Ausgangskonzentration $P_0$ von 96,4 mg/dl lagen die Konzentrationen nach der Perfusion im Mittel bei 1,88 ± 0,23 mg/dl. Damit errechnete sich eine durchschnittliche Clearance von 0,98 ml/Min. bei einem Flow von 1 ml/Min. und eine durchschnittliche prozentuale Elimination von 98,04 % des gesamten Phenobarbitals.

### g) Phenytoin

Die Ausgangskonzentration $P_0$ betrug 50,5 µg/ml. Der Mittelwert $\overline{P}$ der Konzentration nach der Perfusion betrug 0,26 ± 0,27 µg/ml.

Bei einem Flow von 1 ml/Min. errechnete sich eine durchschnittliche Clearance von 0,99 ml/Min. sowie eine durchschnittliche prozentuale Elimination von 99,58 % des Phenytoins.

### Biokompatibilität

Bestimmt wurden die auf Seite 6 genannten Parameter.

### a) Zelluläre Bestandteile des Blutes

Erythrozytenzahl, Hämoglobin und abgeleitete Parameter blieben fast unverändert.

Die Thrombozyten- und Leukozytenzahlen nahmen während der Perfusion ab. Bei den Thrombozyten lag der Abfall zwischen 91,5 und 97,7 % und bei den Leukozyten zwischen 37 und 44,4 % der ursprünglichen Werte.

**b) Nichtzelluläre Bestandteile des Blutes**

Die laborchemischen Parameter blieben im wesentlichen unverändert. Die Schwankungen lagen innerhalb der Standardabweichungen des Labors.

**Patentansprüche**

1. Reversed-Phase-Kieselgel als Mittel zur Hämoperfusion oder Hämoadsorption.

2. Mittel nach Anspruch 1, dadurch gekennzeichnet, daß das Reversed-Phase-Kieselgel einen Porendurchmesser von etwa 10 nm und eine Korngröße von 40 - 60 μm mit einer im wesentlichen unpolaren hydrophoben Oberfläche aufweist.

**Claims**

1. Reversed-phase siliceous gel as preparation for haemoperfusion or haemoadsorption.

2. Preparation according to claim 1 characterised in that the reversed phase siliceous gel has a pore diameter of about 10 nm and a particle size of 40 - 60 μm with a substantially non-polar hydrophobic surface.

**Revendications**

1. Gel de silice en phase inverse en tant qu'agent pour l'hémoperfusion ou l'hémo-adsorption.

2. Agent selon la revendication 1, caractérisé en ce que le gel de silice en phase inverse présente un diamètre de pores d'environ 10 nm et une taille de grains de 40 à 60 μm avec une surface sensiblement non polaire et hydrophobe.